(19) Europäisches Patentamt
European Patent Office
Office européen des brevets

(11) Publication number: **0 430 300 A2**

(12) # EUROPEAN PATENT APPLICATION

(21) Application number: **90123013.6**

(51) Int. Cl.[5]: **C07D 473/00**, A61K 31/52

(22) Date of filing: **30.11.90**

(30) Priority: **01.12.89 JP 313918/89**

(43) Date of publication of application:
**05.06.91 Bulletin 91/23**

(84) Designated Contracting States:
**AT BE CH DE DK ES FR GB GR IT LI LU NL SE Bulletin**

(71) Applicant: **TAKEDA CHEMICAL INDUSTRIES, LTD.**
**3-6, Doshomachi 2-chome Chuo-ku**
**Osaka 541(JP)**

(72) Inventor: **Morimoto, Akira**
**7-8, Ueikeda 1-chome, Ikeda**
**Osaka 563(JP)**
Inventor: **Nishikawa, Kohei**
**5-19 Oharano-kamisatotorimicho,**
**Nishikyo-ku**
**Kyoto 610-11(JP)**

(74) Representative: **Lederer, Franz, Dr. et al**
**Lederer, Keller & Riederer, Patentanwälte,**
**Lucile-Grahn-Strasse 22**
**W-8000 München 80(DE)**

(54) **Xanthine derivatives, their production and use.**

(57) Novel xanthine derivatives of the formula (I):

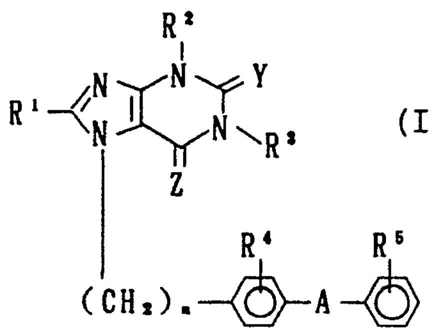

(I)

wherein $R^1$, which may be optionally bound through a hetero atom, is a hydrocarbon residue which may be substituted; one of $R^2$ and $R^3$ is a hydrocarbon residue which may be substituted, and the other is hydrogen or a hydrocarbon residue which may be substituted; $R^4$ is hydrogen, halogen or nitro; $R^5$ is a residue capable of forming an anion or a residue convertible into the anion; A is a direct bond or a spacer having atomic length of two or less; n is an integer of 1 or 2; and Y and Z, which may be the same or different, are an oxygen atom or a sulfur atom; and the pharmaceutically acceptable salts thereof have potent angiotensin II antagonist activity and hypotensive activity, thus being useful as therapeutic agents for treating circulatory system diseases such as hypertensive diseases, heart diseases, strokes, etc.

EP 0 430 300 A2

# XANTHINE DERIVATIVES, THEIR PRODUCTION AND USE

## FIELD OF THE INVENTION

The present invention relates to novel xanthine derivatives having potent pharmacological activity and intermediates for the preparation thereof. More particularly, the present invention relates to compounds having potent angiotensin II antagonist activity and hypotensive activity, which are useful as therapeutic agents for treating circulatory system diseases such as hypertensive diseases, heart diseases, strokes, etc.

## BACKGROUND OF THE INVENTION

The renin-angiotensin system is involved in the homeostatic function to control systemic blood pressure, the volume of body fluid, balance among the electrolytes, etc., associated with the aldosterone system. Development of angiotensin II converting enzyme inhibitors (ACE inhibitor) (this converting enzyme produces angiotensin II which possesses strong vasoconstrictive activity) has clarified the relation between the renin-angiotensin system and hypertension. Since angiotensin II elevates blood pressure via the angiotensin II receptors on cell membranes, angiotensin II antagonists as well as the ACE inhibitor would be useful in treating hypertension.

It has been reported that various angiotensin II analogues such as saralasin, [$Sar^1$ ,$Ile^8$]A II, and the like, possess potent angiotensin II antagonist activity.

It has, however, been reported that, when peptide antagonists are administered parenterally, their actions are not prolonged and, when administered orally, they are ineffective (M. A. Ondetti and D. W. Cushman, Annual Reports in Medicinal Chemistry, 13, 82-91 (1978)).

Non-peptide angiotensin II antagonists are disclosed in Japanese Patent Laid Open No. 71073/1981; No. 71074/1981; No. 92270/1982; No. 157768/1983; No. 240683/1987; No. 23868/1988; and No. 11787/1989, EP Laid Open No. 0323841, etc.

Imidazole derivatives having angiotensin II antagonist activity are disclosed in A. T. Chiu et al., Eur. J. Pharm., 157, 13 (1981), P. C. Wong et al., J. Pharmcol. Exp. Ther., 247, 1 (1988), P. C. Wong et al., Hypertension, 13, 489 (1989), etc.

It has not yet been known that xanthine derivatives possess potent angiotensin II antagonist activity.

## SUMMARY OF THE INVENTION

The present inventors made extensive investigations to prepare useful compounds which have angiotensin II antagonist activity. As a result of these researches, the present inventors have succeeded in synthesizing xanthine derivatives possessing excellently potent angiotensin II antagonist activity and developed their work to accomplish the present invention.

The present invention provides xanthine derivatives having the formula I:

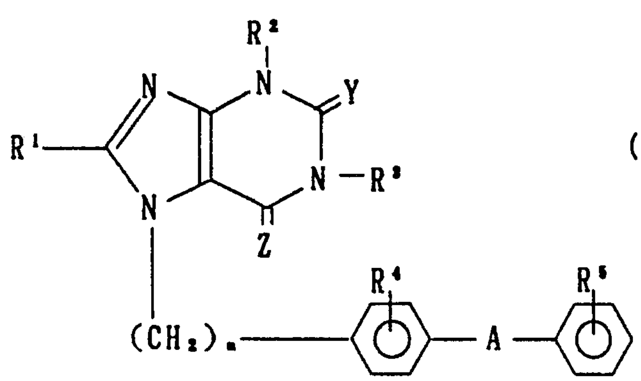

(I)

wherein $R^1$, which may be optionally bound through a hetero atom, is a hydrocarbon residue which may be substituted;

one of $R^2$ and $R^3$ is a hydrocarbon residue which may be substituted, and the other is hydrogen or a hydrocarbon residue which may be substituted;

$R^4$ is hydrogen, halogen or nitro;

$R^5$ is a residue capable of forming an anion or a residue convertible into the anion;

A is a direct bond or a spacer having atomic length of two or less;

n is an integer of 1 or 2; and

Y and Z, which may be the same or different, are an oxygen atom or a sulfur atom;

and the pharmaceutically acceptable salts thereof.

With regard to the foregoing formula (I), hydrocarbon residues for $R^1$ include acyclic hydrocarbon residues such as lower alkyl of 1 to about 8 carbon atoms (e.g. methyl, ethyl, propyl, isopropyl, butyl, isobutyl, sec-butyl, t-butyl, pentyl, i-pentyl, hexyl, heptyl, octyl, and the like), lower alkenyl of 2 to about 8 carbon atoms (e.g. vinyl, allyl, isopropenyl, 2-butenyl, 1 ,3-butadienyl, 2-pentenyl, 2-hexenyl, 2-octenyl, and the like), and lower alkynyl of 2 to about 8 carbon atoms (e.g. ethynyl, 2-propynyl, 2-butynyl, 2-pentynyl, 2-octynyl, and the like); cyclic hydrocarbon residues such as alicyclic hydrocarbon residues of 3 to about 8 carbon atoms (e.g. cyclopropyl, cyclopentyl, cyclohexyl, 2-cyclohexen-1-yl, cyclooctyl, and the like), and aromatic hydrocarbon residues of 6 to about 12 carbon atoms (e.g. phenyl, naphtyl, and the like); and the like.

With regard to the foregoing formula (I), hydrocarbon residues for $R^1$ may be bound to a xanthine nucleus through a hetero atom selected from S, O, and NR wherein R is hydrogen or a hydrocarbon residue which may be substituted and optionally substituted with hydroxyl, lower ($C_1$-$_4$) alkoxy (e.g. methoxy, ethoxy, and the like), lower ($C_1$-$_4$) alkyl (e.g. methyl, ethyl, and the like), halogen (e.g. F, Cl, Br and the like), nitro, amino which may be optionally substituted (e.g. amino, and the like), acyloxy (e.g. lower ($C_1$-$_4$) alkanoyloxy, benzoyloxy and the like), phenyl (e.g. phenyl which may be optionally substituted with halogen, nitro, lower ($C_1$-$_4$) alkoxy, lower ($C_1$-$_4$) alkyl or the like), etc.

Preferred examples of such hydrocarbon residues include lower alkyl of 1 to about 8 carbon atoms which may be bound to a xanthine nucleus through a sulfur atom, lower alkyl of 1 to about 4 carbon atoms substituted with phenyl which may be optionally substituted with halogen, nitro, lower ($C_1$-$_4$) alkoxy, lower ($C_1$-$_4$) alkyl or the like at an optional position on the phenyl ring (e.g. phenyl-lower ($C_1$-$_4$) alkyl such as benzyl, phenethyl, phenylpropyl, and the like) and phenyl which may be optionally substituted with halogen, nitro, lower ($C_1$-$_4$) alkoxy, lower ($C_1$-$_4$) alkyl, or the like at an optional position on the phenyl ring.

Examples of hydrocarbon residues for $R^2$ and $R^3$ include acyclic hydrocarbon residues such as lower alkyl of 1 to about 4 carbon atoms (e.g. methyl, ethyl, propyl, isopropyl, butyl, isobutyl, sec-butyl, t-butyl, and the like), lower alkenyl of 2 to about 4 carbon atoms (e.g. vinyl, allyl, isopropenyl, 2-butenyl, 1,3-butadienyl, and the like), lower alkynyl of 2 to about 4 carbon atoms (e.g. ethynyl, 2-propynyl, 2-butynyl, and the like); cyclic hydrocarbon residues such as alicyclic hydrocarbon residues of 3 to about 6 carbon atoms (e.g. cyclopropyl, cyclopentyl, cyclohexyl, 2-cyclohexen-1-yl, and the like), aromatic hydrocarbon residues of about 6 carbon atoms (e.g. phenyl, and the like); etc.

Said hydrocarbon residues for $R^2$ and $R^3$ may be optionally substituted with hydroxyl, lower ($C_1$-$_4$) alkoxy (e.g. methoxy, ethoxy, and the like), lower ($C_1$-$_4$) alkyl (e.g. methyl, ethyl, and the like), halogen (e.g. F, Cl, Br and the like), nitro, amino which may be optionally substituted (e.g. amino, methylamino, dimethylamino, and the like), acyloxy (e.g. lower ($C_1$-$_4$) alkanoyloxy, benzoyloxy and the like), phenyl (e.g. phenyl which may be optionally substituted with halogen, nitro, lower ($C_1$-$_4$) alkoxy, lower ($C_1$-$_4$) alkyl or the like at an optional position on the phenyl ring), etc.

Among the above-mentioned hydrocarbon residues for $R^2$ and $R^3$, lower ($C_1$-$_4$) alkyl of 1 to about 4 carbon atoms which may be optionally substituted with hydroxyl, lower ($C_1$-$_4$) alkoxy, optionally substituted amino, acyloxy and the like, lower alkyl of 1 to about 4 carbon atoms substituted with phenyl which may be optionally substituted with halogen, nitro, lower ($C_1$-$_4$) alkoxy, lower ($C_1$-$_4$) alkyl or the like at an optional position on the phenyl ring (e.g. phenyl-lower ($C_1$-$_4$) alkyl such as benzyl, phenethyl, phenylpropyl, and the like) and phenyl which may be optionally substituted with halogen, nitro, lower ($C_1$-$_4$) alkoxy, lower ($C_1$-$_4$) alkyl or the like at an optional position on the phenyl ring. More preferred examples of such hydrocarbon residues are lower alkyl of 1 to about 4 carbon atoms.

$R^4$ represents hydrogen, halogen (e.g. Cl, Br and the like), or nitro and may be in the ortho or meta positions. A preferred example of $R^4$ is hydrogen.

Examples of residues capable of forming an anion and residues convertible into the anion for $R^5$ include carboxyl, lower ($C_1$-$_4$) alkoxycarbonyl, cyano, tetrazolyl, trifluoromethanesulfonic amide ($-NHSO_2CF_3$,) , phosphoric acid, sulfonic acid, and the like and these groups are optionally protected with an optionally substituted lower alkyl group or acyl group as shown by $R^6$ and $R^7$ described below. Such residues may include those which are capable of forming anions either chemically or under biological and/or physiological conditions. $R^5$ may be in the ortho, meta or para position, and preferably in the ortho position. More preferred examples of $R^5$ are carboxyl and tetrazolyl.

The compounds wherein $R^5$ is a residue capable of forming an anion or convertible thereinto (e.g. cyano and the like) chemically are useful as synthetic intermediates.

The group A represents a direct bond or a spacer having atomic length of two or less between a phenylene group and a phenyl group. Examples of spacers having atomic length of two or less include divalent chains wherein the constituent number of atoms in a straight chain is 1 or 2 and which may be branched. Examples of such spacers include -C(=O)-, -O-, -S-, -NH-, -C(=O)-NH-, $-O-CH_2-$, $-S-CH_2-$, -CH=CH-, etc.

The groups Y and Z are each an oxygen atom or a sulfur atom and may be the same or different. They are preferably the same and further an oxygen atom.

A preferred embodiment of the invention is a compound of the formula (I')

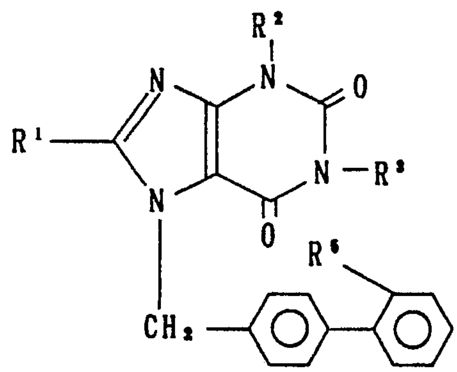

(I')

wherein $R^1$, which may be optionally bound through a sulfur or oxygen atom, preferably a sulfur atom, is lower ($C_{1-8}$) alkyl, phenyl-lower ($C_{1-4}$) alkyl which may be optionally substituted or phenyl which may be optionally substituted;

$R^2$ and $R^3$ are each lower ($C_{1-4}$) alkyl; and

$R^5$ is carboxyl or tetrazolyl;

and the pharmaceutically acceptable salts thereof.

The compounds of the present invention may be prepared by several reaction schemes, as illustrated below for a preferred compound.

Scheme A

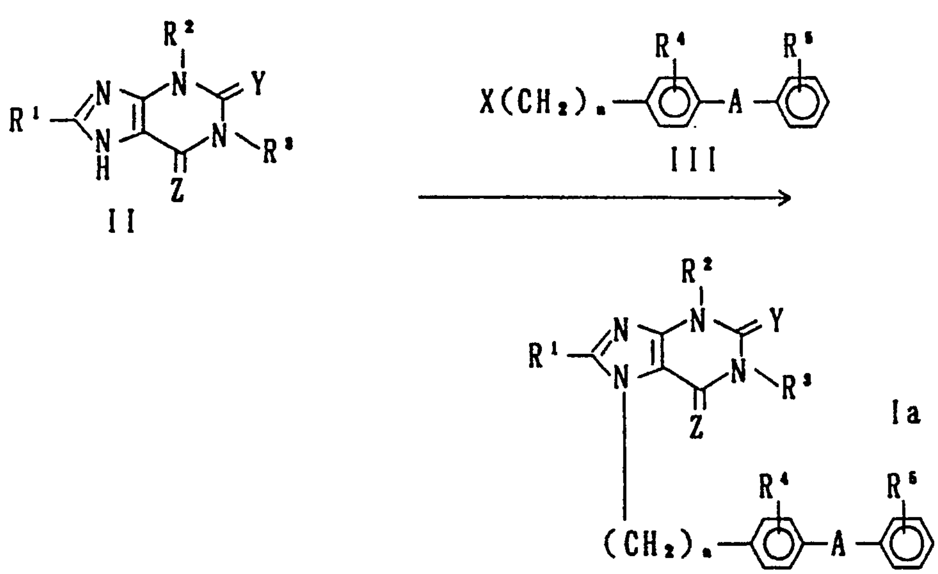

II

III

Ia

wherein $R^1$, $R^2$, $R^3$, $R^4$, $R^5$, Z, Y, A, and n have the above-defined meanings and X is halogen.

Scheme B

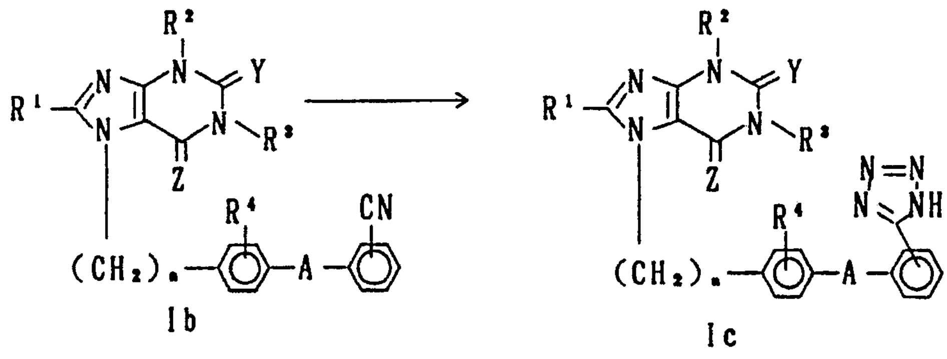

Ib Ic

wherein $R^1$, $R^2$, $R^3$, $R^4$, Z, Y, A, and n have the above-defined meanings.

Scheme C

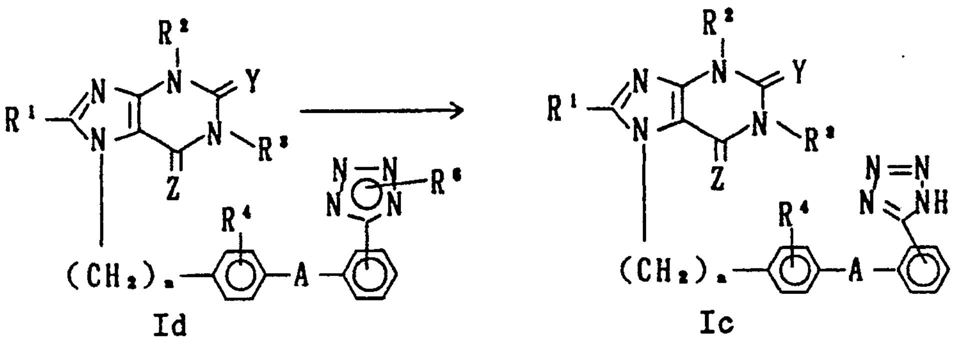

Id Ic

wherein $R^1$, $R^2$, $R^3$, $R^4$, $R^6$, Z, Y, A, and n have the above-defined meanings.

Scheme D

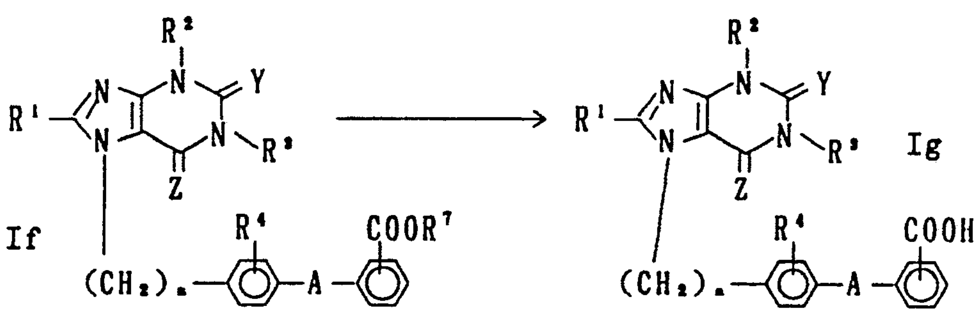

If Ig

wherein $R^1$, $R^2$, $R^3$, $R^4$, $R^7$, Z, Y, A, and n have the above-defined meanings.

The reaction as illustrated in Scheme A is an alkylation using an alkylating agent in the presence of a base. One molar portion of the compound (II) is employed with 1 to 3 moles of the base and about 1 to about 3 moles of the alkylating agent. The reaction is conventionally conducted in solvents such as dimethylformamide, dimethylacetamide, dimethylsulfoxide, acetonitrile, acetone, ethylmethylketone, and the like. Examples of such bases include sodium hydride, potassium t-butoxide, potassium carbonate, sodium carbonate, and the like. Examples of such alkylating agents include substituted halides (e.g. chlorides, bromides, iodides, and the like), substituted sulfonate esters (e.g. methyl p-toluenesulfonate esters, and the like), etc.

The reaction conditions may vary depending on the combination of the base and the alkylating agent. A temperature in the range of from ice-cooling to room temperature is preferred and a reaction period of from about 1 to about 10 hours is preferably employed.

The cyano substituent on the benzene is reacted with various azides to form the tetrazole componds (Ic) as illustrated in Scheme B. One molar portion of the compound (Ib) is employed with about 1 to about 3 moles of the azide. The reaction is conventionally conducted in solvents such as dimethylformamide, dimethylacetamide, toluene, benzene, and the like. Examples of such azides include trialkyl-tin azide, triphenyl-tin azide, hydrogen azide, and the like. In the case wherein the organo-tin azide compound is employed, the reaction is carried out in toluene or benzene by heating under a reflux for a period of from about 10 to about 30 hours. When the hydrogen azide is used, 2 moles of sodium azide and ammonium chloride per compound (Ib) are employed and the reaction is conducted in dimethylformamide at a temperature of from about 100° C to about 130° C for 1 to 3 days. During this reaction, it is preferable to facilitate working by adding an appropriate amount of sodium azide and ammonium chloride.

The reaction as illustrated in Scheme C is used to obtain the compound (Ie) by deprotection of the suitably protected tetrazole derivative (Id). Reaction conditions for the deprotection can vary with the protective group ($R^6$) employed. When $R^6$ is triphenylmethyl, 2-tetrahydropyranyl, methoxymethyl, ethoxymethyl, etc., the reaction is preferably carried out in an aqueous alcohol (e.g. methanol, ethanol, etc.) containing about 0.5 N to about 2 N hydrochloric acid or acetic acid, at room temperature or near for a period from about 1 to about 10 hours.

The reaction as illustrated in Scheme D used is to obtain the compound (Ig) by deprotection of the suitably protected carboxylic acid derivative (If). Deprotection conditions for the reaction can vary with the protective group ($R^7$) employed. When $R^6$ is t-butyl, the reaction is preferably carried out using an excess amount of trifluoroacetic acid in a suitable organic solvent (e.g. chloroform, methylene chloride, acetonitrile, etc.), at room temperature or near for a period from about 1 to about 5 hours.

The compounds (I) thus produced via the reaction processes as depicted in Schemes A, B, C and D can be isolated and purified from the reaction mixture according to conventional methods such as for example, evaporation of solvents, extraction by water or organic solvents, concentration, neutralization, recrystallization, distillation, column chromatography and the like, to obtain a crystalline or oily product.

The compounds (I) of the present invention can be used in the form of salts derived from pharmaceutically or physiologically acceptable acids or bases. These salts include but are not limited to the following: salts with inorganic acids such as hydrochloric acid, sulphuric acid, nitric acid, phosphoric acid and, as the case may be, such organic acids as acetic acid, oxalic acid, succinic acid, maleic acid. Other salts include salts with alkali metals or alkaline earth metals, such as sodium, potassium, calcium or magnesium or with organic bases.

The starting materials (II) may be prepared by or according to methods described in, for example,

(1) D. S. Bariana, Can. J. Chem., 46, 3413 (1968),
(2) A. J. Dietz, $J_R$., and R. M. Burgison, J. Med. Chem., 9, 500 (1966),
(3) K. A. Jacobson, L. Kiriasis, S. Barone, B. J. Bradbury, U. Kammula, J. M. Campagne, S. Secunda, J. W. Daly, J. L. Neumeyer, and W. Pfleiderer, J. Med. Chem., 32, 1873 (1989),
(4) F. F. Blicke and H. C. Godt, Jr., J. Am. Chem. Soc., 76, 2799 (1954),
(5) K. R. H. Wooldridge and R. Slack, J. Chem. Soc., 1962, 1863,
(6) A. J. Dietz, Jr., and R. M. Burgison, J. Med. Chem., 9, 160 (1966), and
(7) Japanese Patent Laid Open No. 247180/1990.

The compounds (IIIa) are prepared by methods described in Japanese Patent Laid Open No. 23868/1988; No. 117876/1989; and EP Laid Open No. 0323841. These compounds (IIIa) can also be easily prepared from the compounds (IV) commercially available or synthesized by known methods, via halogenomethylation as illustrated in Scheme E, according to processes described in, for example, A. A. Vansheidt et al., Khim. Nauka i Prom., 2, 799 (1957), etc. Scheme E

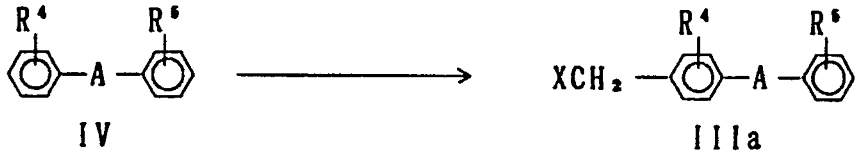

IV

IIIa

wherein each group has the above-defined meaning.

The compounds (IIIb) are prepared from the compounds (IIIa) according to processes as illustrated in Scheme F.

Scheme F

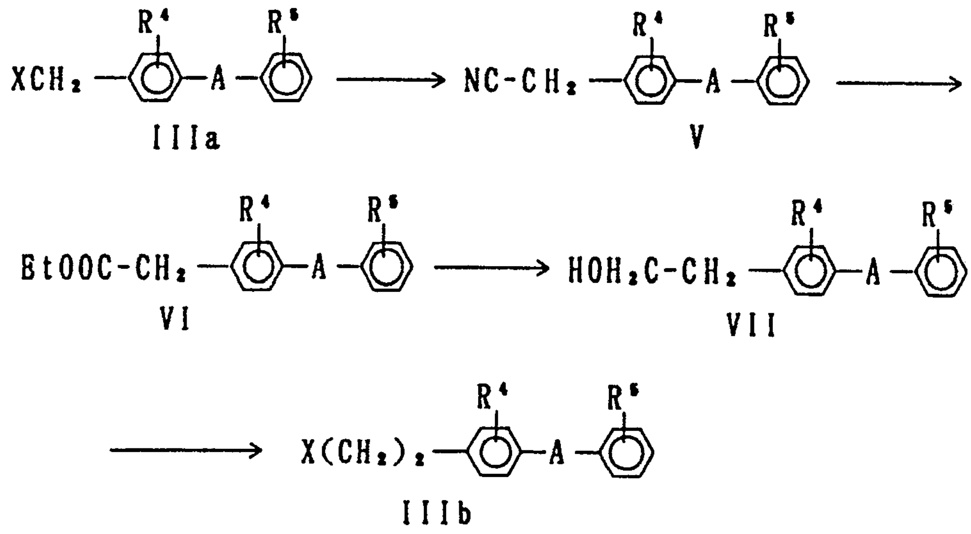

IIIa V VI VII IIIb

wherein each group has the above-defined meaning.

The compounds (I) and salts thereof according to the present invention inhibit strongly vasoconstriction and hypertension derived by angiotensin II and therefore possess potent anti-hypertensive activity in animals, more specifically e.g. humans, dogs, rabbits, rats, etc. Further, the compounds (I) and salts thereof according to the present invention are of quite low toxicity and useful in treating not only hypertension but also circulatory system diseases such as heart diseases, strokes and the like.

For therapeutic use, the compounds (I) and salts thereof can be administered as pharmaceutical compositions (e,g, powders, granules, tablets, pills, capsules, injections, solutions) comprising at least one such compound alone or in admixture with pharmaceutically acceptable carriers, excipients and/or diluents. The pharmaceutical compositions can be formulated in accordance with a conventional method.

Specific dose levels for any particular patient will be employed depending upon a variety of factors including the activity of specific compounds employed, the age, body weight, general health, sex, diet, time of administration, route of administration, rate of excretion, drug combination, and the severity of the particular disease undergoing therapy. When used for treating adult essential hypertension, the active ingredient will preferably be administered in an appropriate amount, for example, selected from the range of from about 10 mg to 100 mg a day orally and from the range of from about 5 mg to 50 mg a day intravenously. The active ingredient will preferably be administered in equal doses two or three times a day.

The foregoing is merely illustrative of the invention and is not intended to limit the invention to the disclosed compounds. Variations and changes which are obvious to one skilled in the art are intended to be within the scope and nature of the invention which are defined in the appended claims.

## Example

The invention is further illustrated but in no way limited by the following working examples, reference examples and biological tests.

### Reference Example 1

A: 6-Amino-5-valerylamino-1,3-dimethyluracil

To a stirred solution of 5,6-diamino-1,3-dimethyluracil hydrate (17.02 g, 0.1 mol) in pyridine (100 ml) was added valerylchloride (13.5 ml, 0.11 mol) and the mixture was allowed to stir at room temperature for 20 hours. To the reaction mixture was then added water and the mixture extracted with ethyl acetate. The ethyl acetate layer was washed with water, dried ($MgSO_4$) , and evaporated under vacuum. The resulting solid residue was triturated with ethyl acetate, and collected by filtration to yield 11.4 g (44.9 %) of the title compound.

IR (Nujol) $cm^{-1}$: 3320, 3170, 1710, 1665, 1640.

NMR (DMSO-$d_6$) $\delta$: 0.87(3H, t, J=7.2Hz), 1.17-1.60(4H, m), 2.23(2H, t, J=7.2Hz), 3.10(3H, s), 6.48(2H, brs), 8.25(1H, brs).

B: 8-n-Butyl-1,3-dimethylxanthine

6-Amino-5-valerylamino-1,3-dimethyluracil (2.54 g, 10 mmoles) obtained in Reference Example 1-A was dissolved in a mixture of 1 N aqueous sodium hydroxide (40 ml) and ethanol (10 ml), and heated at reflux for 1.5 hours with stirring. To the reaction mixture was then added ethyl acetate and the resulting crystals were collected by filtration. The crystals were washed with water and then petroleum to obtain 2.54 g of the crystals. Recrystallization from ethanol gave the title compound (2.27 g, 96.1 %) as a colorless prism.

M.p. 240-241 °C.

IR (Nujol) $cm^{-1}$: 3150, 1715.

NMR ($CDCl_3$)δ: 0.96(3H, t, J=7.4$H_Z$), 1.33-1.54(2H, m), 1.74-1.93(2H, m), 1.78(1H, brs), 2.91(2H, t, J=7.4$H_Z$), 3.48(3H, s), 3.64(3H, s).

Anal. Calc'd for $C_{11}H_{16}N_4O_2$: C, 55.92; H, 6.83; N, 23.71

Found: C, 55.84; H, 6.95; N, 23.56.

Reference Example 2

A: 6-Amino-1,3-dimethyl-5-benzylidinouracil

A mixture of 5,6-diamino-1,3-dimethyluracil (4.56 g, 30 mmoles) in acetic acid (3 ml) and methanol (15 ml) was stirred, and then a solution of benzaldehyde (3.18 g, 30 mmoles) in methanol(10 ml) was added. The mixture was stirred at room temperature for 3 hours. The resulting precipitate was collected by filtration and dried to yield the title compound (5.75 g, 74.2%). The product was recrystallized from dimethylformamide (DMF) and methanol to give 0.36 g of the purified product.

M.p. 229-230 °C.

IR (Nujol) $cm^{-1}$: 3420, 3300, 1695, 1600.

NMR (DMSO-$d_6$) δ: 3.18(3H, S), 3.41(3H, s), 7.29(5H, m), 7.89(2H, dd, J=8.0$H_Z$, 1.8$H_Z$), 9.73(1H, s).

Anal. Calc'd for $C_{13}H_{14}N_4O_2$: C, 60.45; H, 5.46; N, 21.69

Found: C, 60.46; H, 5.45; N, 21.71.

B: 1,3-Dimethyl-8-phenylxanthine

To a solution of 6-amino-1,3-dimethyl-5-benzylidinouracil (5.17 g, 20 mmoles) in ethanol (40 ml) was added ferric chloride ($FeCl_3$) (3.25 g, 20 mmoles), and the mixture was heated at reflux for 4 hours with stirring. The reaction mixture was then allowed to stand at room temperature for 2 days. The resulting precipitate was collected by filtration and washed with ethanol to obtain the title compound (3.59 g, 70.0 %) as a yellow crystalline.

IR (Nujol) $cm^{-1}$: 3160, 1690 1650.

NMR (DMSO-$d_6$) δ: 3.29(3H, S), 3.52(3H, s), 7.28-7.59(5H, m), 7.88(1H, brd, J=4.2$H_Z$).

Anal. Calc'd for $C_{13}H_{12}N_4O_2$: C, 60.93; H, 4.72; N, 21.86

Found: C, 59.50; H, 4.69; N, 22.08.

The following compounds as listed in Table 1 were prepared in the same manner as in Reference Examples 1 and 2, or by or according to methods described in the above-mentioned references (1)-(7).

Table 1a

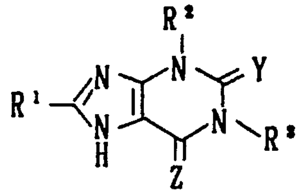

| Ref. Ex. No. | $R^1$ | $R^2$ | $R^3$ | Y | Z | MP. (°C) |
|---|---|---|---|---|---|---|
| 3 | Pr | Me | Me | O | O | 147-148 |
| 4 | Pen | Me | Me | O | O | — |
| 5 | 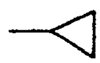 | Me | Me | O | O | — |
| 6 | 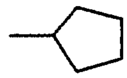 | Me | Me | O | O | 158-159 |
| 7 | $-CH_2Ph$ | Me | Me | O | O | — |
| 8 | $-(CH_2)_3Ph$ | Me | Me | O | O | 206-207 |
| 9 | $-CH_2\underset{Ph}{CH}CH_3$ | Me | Me | O | O | — |
| 10 | Bu | Me | Me | S | O | 206-208 |
| 11 | $-(CH_2)_2-Ph$ | Me | Me | O | O | 191-192 |
| 12 | i-Bu | Me | Me | O | O | 238-239 |
| 13 | Hep | Me | Me | O | O | 196-197 |
| 14 | Dec | Me | Me | O | O | 165-166 |
| 15 | -SBu | Me | Me | O | S | 202-203 |
| 16 | -SBu | Me | Me | S | O | — |
| 17 | -SPr | Me | Me | O | S | — |
| 18 | -SPr | Me | Me | S | O | — |
| 19 | Pr | Pr | Et | O | O | — |

Table 1a (continued)

| Ref. Ex. No. | $R^1$ | $R^2$ | $R^3$ | Y | Z | MP. (°C) |
|---|---|---|---|---|---|---|
| 20 | Bu | Pr | Et | O | O | 116-117 |
| 21 | Bu | Pr | Pr | O | O | 157-158 |
| 22 | Bu | Me | Pr | O | O | 166-167 |
| 23 | -SBu | Pr | Pr | O | O | 112-113 |
| 24 | -SBu | Pr | Et | O | O | 111-112 |
| 25 | Bu | Bu | Bu | O | O | 135-136 |
| 26 | Bu | Bu | Pr | O | O | 140-141 |
| 27 | Bu | Me | Bu | O | O | 174-175 |
| 28 | $-CH_2CH=CHCH_2CH_3$ | Pr | Pr | O | O | 139-141 |
| 29 | Bu | Et | Et | O | O | 151-152 |
| 30 | Bu | Me | Me | O | O | 240-241 |
| 31 | -SBu | Me | Me | O | O | — |
| 32 | OEt | Me | Me | O | O | 289-290 |
| 33 | OEt | Et | Et | O | O | 181-182 |

Table 1b

| Ref. Ex. No. | IR(Nujol) $cm^{-1}$ | NMR (DMSO-$d_6$) δ |
|---|---|---|
| 3 | 3180,1720,1630 | 0.90(3H,t),1.64-1.84(2H,m),2.66(2H,t),3.23(3H,s),3.42(3H,s) |
| 4 | 3180,1720,1030 | 0.87(3H,t),1.17-1.41(4H,m),1.62-1.79(2H,m),2.67(2H,t),3.23(3H,s),3.42(3H,s) |
| 5 | 3150,3050,1705 1660 | 0.93-1.15(4H,m),1.93-2.08(1H,m),3.23(3H,s),3.38(3H,s) |
| 6 | 3220,1725,1640 | 1.54-2.09(8H,m),3.16(1H,t),3.24(3H,s),3.43(3H,s) |
| 7 | 3400,3300,3220 1720,1680,1635 | 3.23(3H,s),3.41(3H,s),4.05(2H,s),7.15-7.40(5H,m) |
| 8 | 3180,1720,1635 | 2.00(2H,dt),2.63(2H,t),2.71(2H,t),3.23(3H,s),3.43(3H,s),7.13-7.35(5H,m) |
| 9 | 3150,1700,1650 | 1.12(3H,d),2.63-3.01(2H,m),3.22(3H,s),3.43(3H,s),7.12-7.36(5H,m) |
| 10 | 3125,1690,1595 | 0.90(3H,t),1.32(2H,m),1.70(2H,m),2.74(2H,t),3.69(3H,s),3.87(3H,s) |
| 11 | 3140,1715,1645 | 1.51-1.81(4H,m),2.60(2H,t),2.68(2H,t),3.23(3H,s),3.41(3H,s),7.10-7.32(5H,m) |
| 12 | 3170,1710,1630 | 0.88(3H,s),0.92(3H,s),2.02(1H,m),2.56(2H,d),3.24(3H,s),3.43(3H,s) |
| 13 | 3170,1715,1625 | 0.88-0.93(3H,m),1.12-1.41(8H,m),1.59-1.78(2H,m),2.67(2H,t),3.23(3H,s),3.42(3H,s) |
| 14 | 3155,1715,1645 1600 | 0.85(3H,t),1.13-1.39(14H,m),1.60-1.79(2H,m),2.67(2H,t),3.23(3H,s),3.42(3H,s) |
| 15 | 3160,1655,1600 | 0.96(3H,t),1.46(2H,m),1.75(2H,m),3.28(2H,t),3.63(3H,s),3.83(3H,s) |

Table 1b (continued)

| Ref. Ex. No. | IR(Nujol) $cm^{-1}$ | NMR (DMSO-$d_6$) δ |
|---|---|---|
| 16 | 3100,1685,1595 | 0.91(3H,t),1.40(2H,m),1.67(2H,m), 3.25(2H,t),3.67(3H,s),3.85(3H,s) |
| 17 | 3215,1645,1615 1595 | 0.99(3H,t),1.63-1.83(2H,m),3.25(2H, t),3.47(3H,s),3.66(3H,s) |
| 18 | 3100,3050,1685 1675,1595 | 0.99(3H,t),1.63-1.83(2H,m),3.23(2H, t),3.36(3H,s),3.67(3H,s),3.85(3H,s) |
| 19 | 3135,3075,1695 1655 1595 | 0.88(3H,t),0.91(3H,t),1.12(3H,t), 1.64-1.81(4H,m),2.66(2H,t),3.80-4.12 (4H,m) |
| 20 | 3145,1700,1655 1600 | 0.88(3H,t),0.89(3H,t),1.12(3H,t), 1.18-1.42(2H,m),1.50-1.77(4H,m),2.69 (2H,t),3.80-4.10(4H,m) |
| 21 | 3145,1705,1650 | 0.95(3H,t),0.98(3H,t),1.00(3H,t), 1.33-1.54(2H,m),1.63-1.95(6H,m),2.88 (2H,t),4.05(2H,t),4.11(2H,t) |
| 22 | 3140,1715,1650 1600 | 0.87(3H,t),0.89(3H,t),1.22-1.41(2H, m),1.46-1.76(4H,m),2.68(2H,t),3.42 (3H,s),3.82(2H,t) |
| 23 | 3110,1750,1655 1600 | 0.94(3H,t),0.97(3H,t),0.99(3H,t), 1.38-1.59(2H,m),1.66-1.94(6H,m),3.27 (2H,t),4.07(4H,q) |
| 24 | 3120,1700,1660 1600 | 0.94(3H,t),0.98(3H,t),1.31(3H,t), 1.38-1.60(2H,m),1.66-1.93(4H,m),3.27 (2H,dq),4.08(2H,q),4.16(2H,q) |
| 25 | 3130,1700,1650 1600 | 0.87(3H,t),0.91(3H,t),0.94(3H,t), 1.20-1.70(12H,m),2.68(2H,t),3.87 (2H,t),3.97(2H,t) |
| 26 | 3130,1700,1650 1600 | 0.97(3H,t),0.98(3H,t),1.24-1.93(10H, m),2.86(2H,t),4.02(2H,t),4.14(2H,t) |
| 27 | 3140,1700,1650 1605 | 0.96(3H,t),0.97(3H,t),1.42(4H,m), 1.62-1.94(9H,m),2.88(2H,t),3.63(3H, s),4.08(2H,t) |

Table 1b (continued)

| Ref. Ex. No. | IR(Nujol) $cm^{-1}$ | NMR ($DMSO-d_6$) δ |
|---|---|---|
| 28 | 3140,1705,1655 1600 | 0.98(6H,t),1.62-2.13(6H,m),3.57(2H, d),3.99-4.18(4H,m),5.68(1H,t),5.71 (1H,t) |
| 29 | 3140,1700,1655 1600 | 0.96(3H,t),1.30(3H,t),1.34(3H,t), 1.20-1.55(2H,m),1.83(2H,m),2.88(2H, t),4.15(2H,q),4.21(2H,q) |
| 30 | 3150,1715, | 0.96(3H,t),1.33-1.54(2H,m),1.74-1.93 (2H,m),2.91(2H,t),3.48(3H,s),3.64 (3H,s) |
| 31 | 3130,1715,1630 | 0.90(3H,t),1.40(2H,m),1.65(2H,m), 3.19(2H,t),3.23(3H,s),3.42(3H,s) |
| 32 | 3160,1710,1660 1615 | 1.36(3H,t),3.22(3H,s),3.38(3H,s), 4.43(2H,q) |
| 33 | 3160,1705,1660 1615 | 1.28(3H,t),1.34(3H,t),1.44(3H,t), 4.13(4H,q),4.51(2H,q) |

Reference Example 34

8-n-Butyl-7-(2'-cyanobiphenyl-4-yl)methyl-1,3-dimethylxanthine

Sodium hydride (oily, 60%)(0.22 g, 5.5 mmoles) was suspended in N,N-dimethylformamide (DMF)(15 ml) and the mixture was stirred under ice-cooling. To the mixture was added a solution of 8-n-butyl-1,3-dimethylxanthine (1.18 g, 5.0 mmoles) in DMF (15 ml) and the mixture stirred for 30 minutes under ice-cooling. A solution of 4-(2-cyanophenyl)benzylbromide (1.50 g, 5.5 mmoles) in DMF (10 ml) was added dropwise to this mixture. After stirring for 1 hour under ice-cooling, the reaction mixture was poured into water and extracted with ethyl acetate. The ethyl acetate layer was washed with water, dried ($MgSO_4$) , and evaporated in vacuo. The resulting residue was subjected to column chromatography employing silica gel and eluted with n-hexane-ethyl acetate (1:4 v/v). The resulting fractions containing the product were collected and evaporated in vacuo. The crystalline residue was recrystallized from dichloromethane and ethyl acetate to give the title compound (1.68 g, 78.6 %) as a white crystalline.

M.p. 165-166 °C.

IR (Nujol) $cm^{-1}$: 2250, 1710, 1660.

NMR ($CDCl_3$)δ: 0.91(3H, t, J=7.2Hz), 1.29-1.48(2H, m), 1.61-1.78(2H, m), 2.73(2H, t, J=7.2Hz), 3.42(3H, s), 3.62(3H, s), 5.63(2H, s), 7.29(2H, d, J=7.0Hz), 7.40-7.81(6H, m).

Anal. Calc'd for $C_{25}H_{25}N_5O_2$: C, 70.24; H, 5.89; N, 16.38

Found: C, 70.23; H, 5.87; N, 16.18.

Reference Example 35

7-(2'-t-Butoxycarbonylbiphenyl-4-yl)methyl-8-n-butyl-1,3-dimethylxanthine

To an ice-cooled, stirred mixture of sodium hydride (60% dispersion in mineral oil)(90 mg, 2 mmole) in DMF(8 ml) was added a solution of 8-n-butyl-1,3-dimethylxanthine (0.43 g, 1.8 mmoles) in DMF (8 ml). After stirring for 20 minutes under cooling, a solution of t-butyl 4'-bromomethylbiphenyl-2-carboxylate (0.95 g, 2.7 mmol) in DMF (6 ml) was added to this mixture and the mixture was stirred at room temperature for one hour. The reaction mixture was poured into water and extracted with ethyl acetate. The organic layer was washed with water, dried ($MgSO_4$) , and evaporated in vacuo. The resulting oil was subjected to column

chromatography employing silica gel and eluted with n-hexane-ethyl acetate (1:4 v/v). The resulting fractions containing the product were evaporated in vacuo to obtain a crystalline product. The product was recrystallized from ethyl acetate-ether to give the title compound (0.72 g, 78.7 %) as a colorless prism.

M.p. 155-156 °C.

IR (Nujol) $cm^{-1}$: 1710, 1700, 1660.

NMR ($CDCl_3$)δ: 0.94(3H, t, J=7.2Hz), 1.22(9H, s), 1.31-1.52(2H, m), 1.64-1.81(2H, m), 2.73(2H, t, J=7.2Hz), 3.41(3H, s), 3.61(3H, s), 5.60(2H, s), 7.17-7.53(7H, m), 7.75-7.87(1H, m).

Anal. Calc'd for $C_{29}H_{34}N_4O_4$: C, 69.30; H, 6.82; N, 11.15

Found: C, 68.91; H, 6.93; N, 10.94.

Reference Example 36

8-Ethoxy-1,3-dimethyl-7-[[2'-(N-triphenylmethyl-tetrazole-5-yl)biphenyl-4-yl]methyl]xanthine

To a solution of 8-ethoxy-1,3-dimethylxanthine (0.90 g) in DMF (15 ml) was added sodium hydride (60% dispersion in mineral oil, 0.23 g). After stirring at room temperature for 15 minutes, a solution of [2'-(N-triphenylmethyltetrazole-5-yl)biphenyl-4-yl]methyl-bromide (2.51 g) in DMF (15 ml) was added to this mixture, and the mixture was stirred at room temperature for three hours followed by addition of water and extraction with chloroform. The extract was washed with water, dried ($MgSO_4$) , and evaporated in vacuo. The resulting residue was chromatographed on a silica gel column to give a colorless syrup. The product was crystalized from ethyl acetate-ether to afford 1.29 g (41 %) of the title compound as a colorless crystal.

M.p. 168-169 °C.

Anal. Calc'd for $C_{42}H_{36}N_8O_3$: C, 71.98; H, 5.18; N, 15.99

Found: C, 71.98; H, 5.27; N, 15.72.

The following compounds as listed in Table 2 were prepared in the same manner as in Reference Examples 34, 35, and 36.

Table 2a

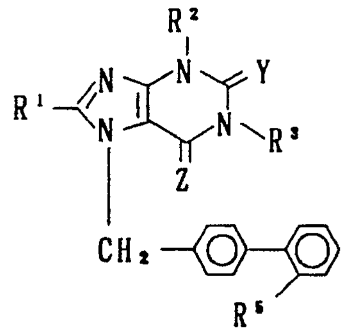

| Ref. Ex. No. | $R^1$ | $R^2$ | $R^3$ | $R^5$ | Y | Z | Yield, MP. |
|---|---|---|---|---|---|---|---|
| 37 | Pr | Me | Me | CN | O | O | 95 % 147-148°C |
| 38 | Pen | Me | Me | CN | O | O | 91 % 77-78°C |
| 39 | Ph | Me | Me | CN | O | O | 86 % 114-116°C |
| 40 | 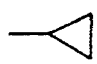 | Me | Me | CN | O | O | 96 % 181-182°C |
| 41 | 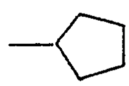 | Me | Me | CN | O | O | 90 % 158-159°C |
| 42 | $-CH_2Ph$ | Me | Me | CN | O | O | 63 % 186-187°C |
| 43 | $-(CH_2)_3Ph$ | Me | Me | CN | O | O | 97 % 120-121°C |
| 44 | $-CH_2CHCH_3$<br>Ph | Me | Me | CN | O | O | 87 % Amorphous |
| 45 | Bu | Me | Me | CN | S | O | 93 % 184-185°C |
| 46 | $-(CH_2)_4Ph$ | Me | Me | CN | O | O | 97 % 117-118°C |
| 47 | iso-Bu | Me | Me | CN | O | O | 96 % 130-131°C |

Table 2a (continued)

| Ref. Ex. No. | $R^1$ | $R^2$ | $R^3$ | $R^5$ | Y | Z | Yield, MP. |
|---|---|---|---|---|---|---|---|
| 48 | Hep | Me | Me | CN | O | O | 99 % 103-104°C |
| 49 | -SBu | Me | Me | Tet-Tri | O | S | 61 % 146-147°C |
| 50 | -SBu | Me | Me | Tet-Tri | S | O | 58 % Oil |
| 51 | -SPr | Me | Me | Tet-Tri | O | S | 53 % 135-136°C |
| 52 | -SPr | Me | Me | Tet-Tri | S | O | 61 % 164-165°C |
| 53 | Pr | Pr | Et | CN | O | O | 97 % Syrup |
| 54 | Bu | Pr | Et | Tet-Tri | O | O | 63 % Amorphous |
| 55 | Bu | Pr | Pr | Tet-Tri | O | O | 65 % 149-150°C |
| 56 | Bu | Me | Pr | Tet-Tri | O | O | 59 % 199-200°C |
| 57 | -SBu | Pr | Pr | Tet-Tri | O | O | 85 % 115-116°C |
| 58 | -SBu | Pr | Et | Tet-Tri | O | O | 85 % 70-73°C |

Table 2a (continued)

| Ref. Ex. No. | $R^1$ | $R^2$ | $R^3$ | $R^6$ | Y | Z | Yield, MP. |
|---|---|---|---|---|---|---|---|
| 59 | Bu | Bu | Bu | Tet-Tri | O | O | 76 % 91-92°C |
| 60 | Bu | Bu | Pr | Tet-Tri | O | O | 64 % 86-87°C |
| 61 | Bu | Me | Bu | Tet-Tri | O | O | 75 % 198-199°C |
| 62 | $-CH_2CH=CHCH_2CH_3$ | Pr | Pr | Tet-Tri | O | O | 40 % |
| 63 | Bu | Et | Et | Tet-Tri | O | O | 78 % Amorphous |
| 64 | Pen | Me | Me | COOtBu | O | O | 82 % 133-134°C |
| 65 | Bu | Me | Me | CN | S | O | 93 % 184-185°C |
| 66 | Dec | Me | Me | COOtBu | O | O | 92 % 103-104°C |
| 67 | SBu | Me | Me | COOtBu | O | O | 87 % Syrup |
| 68 | SBu | Me | Me | Tet-Tri | O | O | 60 % 87-90°C |
| 69 | OEt | Et | Et | Tet-Tri | O | O | 90 % 119-121°C |

Table 2b

| Ref. Ex. No. | IR(Nujol) $cm^{-1}$ | NMR ( δ ) |
|---|---|---|
| 37 | 2225,1710,1655 | 0.99(3H,t),1.77(2H,dt),2.72(2H,t), 3.42(3H,s),3.62(3H,s),5.63(2H,s) 7.26-7.79(8H,m) |
| 38 | 2220,1705,1660 | 0.88(3H,t),1.26-1.40(4H,m),1.68-1.82 (2H,m),2.73(2H,t),3.42(3H,s),3.62 (3H,s),5.62(2H,s),7.26(2H,s),7.28 (2H,d),7.40-7.70(4H,m) |
| 39 | 2220,1700,1660 | 3.43(3H,s),3.68(3H,s),5.71(2H,s), 7.15(1H,s),7.19(1H,s),7.26(1H,s), 7.39-7.70(9H,m),7.75(1H,d) |
| 40 | 2215,1695,1670 1660 | 1.03-1.27(4H,m),1.84-1.99(1H,m),3.40 (3H,s),3.55(3H,s),5.71(2H,s),7.27 (1H,s),7.35-7.59(5H,m),7.65(1H,t), 7.76(1H,d) |
| 41 | 2220,1700,1660 | 1.55-2.03(8H,m),3.15(1H,t),3.41(3H, s),3.61(3H,s),5.66(2H,s),7.23-7.32 (2H,m),7.40-7.69(5H,m),7.76(1H,d) |
| 42 | 2220,1705,1655 | 3.41(3H,s),3.65(3H,s),4.14(2H,s), 5.50(2H,s),7.12-7.37(7H,m),7.41-7.55 (4H,m),7.65(1H,dt),7.77(1H,dd) |
| 43 | 2220,1710,1650 1610 | 2.07(2H,m),2.67-2.80(4H,m),3.42(3H, s),3.61(3H,s),5.50(2H,s),7.10-7.35 (7H,m),7.40-7.52(4H,m),7.64(1H,t), 7.75(1H,d) |
| 44 | 2220,1700,1655 1605 | 1.37(3H,d),2.97(2H,d),3.38(3H,s), 3.40(1H,t),3.63(3H,s),5.19(2H,dd), 7.11-7.36(7H,m),7.39-7.53(4H,m),7.63 (1H,t),7.75(1H,d) |
| 45 | 2205,1690,1590 | 0.92(3H,t),1.39(2H,m),1.72(2H,m), 2.76(2H,t),3.85(3H,s),4.04(3H,s), 5.65(2H,s),7.21-7.32(2H,m),7.40-7.59 (4H,m),7.65(1H,dt),7.76(1H,d) |

Table 2b (continued)

| Ref. Ex. No. | IR(Nujol) $cm^{-1}$ | NMR ( δ ) |
|---|---|---|
| 46 | 2210,1695,1650 1600 | 1.72-2.03(4H,m),2.62(2H,t),2.74(2H,t),3.41(3H,s),3.61(3H,s),5.58(2H,s),7.09-7.64(11H,m),7.65(1H,dt),7.76 (1H,dd) |
| 47 | 2210,1695,1650 1600 | 0.86(3H,s),0.90(3H,s),1.97-2.16(1H,m),2.63(2H,d),3.24(3H,s),3.46(3H,s),5.67(2H,s),7.32(2H,d),7.52-7.60(4H,m),7.77(1H,dt),7.91(1H,d) |
| 48 | 2210,1695,1660 1590 | 0.86(3H,t),1.14-1.46(8H,m),1.60-1.80 (2H,m),2.73(2H,t),3.42(3H,s),3.62 (3H,s),5.62(2H,s),7.26-7.61(6H,m),7.76(1H,d) |
| 49 | 1675,1585 | 0.86(3H,t),1.43(2H,m),1.77(2H,m),3.24(2H,t),3.51(3H,s),3.71(3H,s),5.17(2H,s),6.88-7.01(6H,m),7.05-7.57 (16H,m),7.96(1H,dd) |
| 50 | 1690,1600 | 0.95(3H,t),1.35-1.56(2H,m),1.64-1.81 (2H,m),3.28(2H,t),3.81(3H,s),3.99 (3H,s),5.36(2H,s),6.85-7.54(22H,m),7.86-7.94(1H,m) |
| 51 | 1680,1595 | 1.00(3H,t),1.73-1.92(2H,m),3.22(2H,t),3.52(3H,s),3.71(3H,s),5.17(2H,s),6.86-7.03(6H,m),7.05-7.58(16H,m),7.93-8.00(1H,m) |
| 52 | 1690,1600 | 0.92(3H,t),1.60-1.80(2H,m),3.22(2H,t),3.65(3H,s),3.88(3H,s),5.41(2H,s),6.78-6.92(6H,m),7.03-7.67(16H,m),7.79(1H,dd) |
| 53 | — | 0.98(3H,t),0.99(3H,t),1.25(3H,t),1.64-1.93(4H,m),2.71(2H,t),3.96-4.23 (4H,m),5.62(2H,s),7.26-7.72(7H,m),7.76(1H,d) |

Table 2b (continued)

| Ref. Ex. No. | IR(Nujol) $cm^{-1}$ | NMR ( $\delta$ ) |
|---|---|---|
| 54 | 1695,1655,1600 | 0.85(3H,t),0.87(3H,t),1.11(3H,t), 1.19-1.45(4H,m),1.49-1.81(4H,m), 3.10-3.26(2H,m),3.78-4.13(4H,m),5.37 (2H,s),6.79-7.66(22H,m),7.78(1H,dd) |
| 55 | 1700,1670,1600 | 0.88(3H,t),0.95(3H,t),0.98(3H,t), 1.22-1.43(2H,m),1.55-1.92(6H,m), 2.58(2H,t),3.96(2H,t),4.07(2H,t), 5.45(2H,s),6.89-7.55(22H,m),7.91(1H, dd) |
| 56 | 1695,1660,1600 | 0.88(3H,t),0.95(3H,t),1.22-1.43(2H, m),1.54-1.81(4H,m),2.59(2H,t),3.59 (3H,s),3.96(2H,t),5.45(2H,s),6.88-7.62(22H,m),7.87-7.97(1H,m) |
| 57 | 1700,1665,1600 | 0.86(3H,t),0.87(3H,t),0.89(3H,t), 1.24-1.43(2H,m),1.48-1.81(6H,m),3.18 (2H,t),3.82(2H,t),3.96(2H,t),5.37 (2H,s),6.78-7.66(22H,m),7.78(1H,d) |
| 58 | 1700,1660,1600 | 0.85(3H,t),0.87(3H,t),1.11(3H,t), 1.19-1.45(4H,m),1.49-1.81(4H,m),3.10 -3.26(2H,m),3.78-4.13(4H,m),5.37(2H, s),6.79-7.66(22H,m),7.78(1H,dd) |
| 59 | 1695,1660,1600 | 0.87(3H,t),0.94(3H,t),0.96(3H,t), 1.21-1.86(12H,m),2.57(2H,t),3.99 (2H,t),4.10(2H,t),5.44(2H,s),6.85-7.55(22H,m),7.90(1H,dd) |
| 60 | 1700,1660,1600 | 0.88(3H,t),0.96(6H,t),1.22-1.86(8H, m),2.58(2H,t),3.95(2H,t),4.10(2H,t), 5.45(2H,s),6.80-7.55(22H,m),7.90(1H, dd) |
| 61 | 1695,1655,1600 | 0.87(3H,t),0.94(3H,t),1.22-1.51(4H, m),1.53-1.74(4H,m),2.58(2H,t),3.59 (3H,s),4.00(2H,t),5.45(2H,s),6.89-7.56(22H,m),7.87-7.96(1H,m) |

Table 2b (continued)

| Ref. Ex. No. | IR(Nujol) $cm^{-1}$ | NMR (δ) |
|---|---|---|
| 62 | —— | 0.92(3H,t),0.94(3H,t),0.99(3H,t), 1.44-1.97(6H,m),2.17(2H,m),3.95(2H, t),4.10(2H,t),5.48(2H,s),6.72-7.57 (22H,m),7.80-7.96(1H,m) |
| 63 | 1700,1650,1600 | 0.88(3H,t),1.25(3H,t),1.36(3H,t), 1.33(2H,m),1.61(2H,m),2.59(2H,t), 4.07(2H,q),4.18(2H,q),5.46(2H,s), 6.88-7.54(25H,m),7.88-7.94(1H,m) |
| 64 | 1705,1660 | 0.86-0.97(3H,m),1.22(9H,s),1.28-1.40 (4H,m),1.66-1.84(2H,m),2.72(2H,t), 3.41(3H,s),3.61(3H,s),5.60(2H,s), 7.17-7.53(7H,m),7.78(1H,dd) |
| 65 | 2205,1690,1590 | 0.92(3H,t),1.39(2H,m),1.72(2H,m), 2.76(2H,t),3.85(3H,s),4.04(3H,s), 5.65(2H,s),7.21-7.32(2H,m),7.40-7.59 (4H,m),7.65(1H,dt),7.76(1H,d) |
| 66 | 1710,1700,1650 1595 | 0.87(3H,t),1.10-1.45(14H,m),1.68-1.82(2H,m),2.72(2H,t),3.41(3H,s), 3.61(3H,s),5.60(2H,s),7.15-7.53(7H, m),7.78(1H,dd) |
| 67 | 1700,1660,1605 | 0.96(3H,t),1.18(9H,s),1.47(2H,m), 1.75(2H,m),3.30(2H,t),3.39(3H,s), 3.57(3H,s),5.50(2H,s),7.23-7.52(7H, m),7.76(1H,dd) |
| 68 | 1700,1655,1600 | 0.94(3H,t),1.44(2H,m),1.71(2H,m), 3.25(2H,t),3.39(3H,s),3.57(3H,s), 5.35(2H,s),6.91-7.54(22H,m),7.89(1H, dd) |
| 69 | 1700,1665,1610 | 1.21(3H,t),1.25(3H,t),1.42(3H,t), 4.07(2H,q),4.10(2H,q),4.51(2H,q), 5.19(2H,s),6.89-7.54(22H,m),7.86-7.93(1H,m) |

Working Example 1

8-n-Butyl-7-(2'-((1H-tetrazole-5-yl)biphenyl-4-yl) methyl)-1,3-dimethylxanthine

To a solution of 8-n-butyl-7-(2'-cyanobiphenyl-4-yl)methyl-1,3-dimethylxanthine (1.26 g, 2.9 mmoles) in DMF (50 ml) were added 1.98 g of sodium azide (30 mmoles) and 1.63 g of ammonium chloride (30 mmoles), and the mixture was stirred at 115° C for 7 days. The reaction mixture was poured into water and extracted with ethyl acetate. The ethyl acetate layer was washed with water, dried ($MgSO_4$), and evaporated in vacuo. The resulting oil was subjected to column chromatography employing silica gel and eluted with

ethyl acetate. The resulting fractions containing the product were collected and evaporated *in vacuo*. The crystalline residue was recrystallized from isopropyl ether to give the title compound (0.83 g, 59.8 %) as a white crystalline.

M.p. 194-195° C.

IR (Nujol) $cm^{-1}$: 1700, 1655.

NMR ($CDCl_3$)δ: 0.90(3H, t, J=7.0Hz), 1.28-1.48(2H, m), 1.60-1.79(2H, m), 2.70(2H, t, J=7.2Hz), 3.28(3H, s), 3.53(3H, s), 5.49(2H, s), 7.09(4H, dd, J=8.2Hz, J=13Hz), 7.37-7.64(3H, m), 7.96(1H, dd, J=1.0Hz,6.2Hz).

Anal. Calc'd for $C_{25}H_{26}N_8O_2$: C, 63.82; H, 5.57; N, 23.81

Found: C, 63.69; H, 5.68; N, 23.69.

Working Example 2

8-Ethoxy-1,3-dimethyl-7-[[2'-(1H-tetrazole-5-yl)-biphenyl-4-yl)methyl)xanthine

A mixture of 8-ethoxy-1,3-dimethyl-7-[[2'-(N-triphenylmethyltetrazole-5-yl)biphenyl-4-yl]methyl]xanthine (1.18 g) in 1 N HCl (5 ml) and methanol (20 ml) was allowed to stir at room temperature for 6 hours. The reaction mixture was extracted with chloroform and the organic layer was washed with water and dried ($MgSO_4$). The solvent was evaporated *in vacuo* to give a syrup, which was purified by silica gel column chromatography to give a syrup. The syrup was crystallized from ethyl acetate to give 0.69 g (89 %) of the title compound as a colorless crystal.

M.p. 224-225° C.

Anal. Calc'd for $C_{23}H_{22}N_8O_3 \bullet 0.2H_2O$ : C, 60.25; H, 4.84; N, 24.44

Found: C, 59.98; H, 4.95; N, 23.80.

NMR (DMSO-$d_6$)δ: 1.34(3H, t), 3.22(3H, s), 3.39(3H, s), 4.50(2H, q), 5.23(2H, s), 7.07(2H, d), 7.23(2H, d), 7.49-7.73(4H, m).

IR (Nujol) $cm^{-1}$: 1710, 1660, 1620.

Working Example 3

8-n-Butyl-7-(2'-carboxybiphenyl-4-yl)methyl-1,3-dimethylxanthine

A mixture of 7-(2'-t-butoxycarbonylbiphenyl-4-yl)methyl-8-n-butyl-1,3-dimethylxanthine (0.40 g, 0.8 mmoles) in trifluoroacetic acid (3 ml) was stirred for one hour under ice-cooling. The reaction mixture was concentrated *in vacuo*. The resulting residue was recrystallized from ether to give the title compound (0.31 g, 87.2 %) as a colorless prism.

M.p. 181-182° C.

IR (Nujol) $cm^{-1}$: 1700, 1650.

NMR (DMSO-$d_6$) δ: 0.82(3H, t, J=7.2Hz), 1.19-1.42(2H, m), 1.49-1.67(2H, m), 2.70(2H, t, J=7.2Hz), 3.23-(3H, s), 3.43(3H, s), 5.60(2H, s), 7.18-7.59(7H, m), 7.71 (1H, dd, J=1.4Hz,7.6Hz).

Anal. Calc'd for $C_{25}H_{26}N_4O_6$: C, 66.71; H, 5.91; N, 12.45

Found: C, 66.79; H, 5.87; N, 12.25.

Working Example 4

7-(2'-Carboxybiphenyl-4-yl)methyl-8-n-pentyl-1,3-dimethylxanthine

The title compound was prepared from 8-n-pentyl-1,3-dimethylxanthine in the same manner as in Reference Example 35 and Working Example 3.

M.p. 142-143° C.

IR (Nujol) $cm^{-1}$: 1700, 1655.

NMR (DMSO-$d_6$) δ: 0.75-0.90(3H, m), 1.12-1.36(4H, m), 1.50-1.70(2H, m), 2.71(2H, t, J=7.6Hz), 3.25(3H, s), 3.34(3H, brs), 3.45(3H, s), 5.62(2H, s), 7.22(2H, d J=8.4Hz), 7.30(2H, d, J=8.4Hz), 7.31-7.61(3H, m), 7.72(1H, dd, J=1.4Hz, 7.6Hz).

Anal. Calc'd for $C_{26}H_{28}N_4O_4$: C, 67.81 ; H, 6.13; N, 12.17

Found: C, 67.66; H, 6.14; N, 12.07.

The following compounds as listed in Table 3 were prepared in the same manner as in Working Examples 1-4.

Table 3a

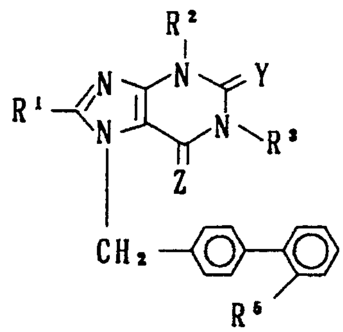

| Working Example No. | $R^1$ | $R^2$ | $R^3$ | $R^5$ | Y | Z | Yield, MP. |
|---|---|---|---|---|---|---|---|
| 5 | Pr | Me | Me | Tet | O | O | 59 %<br>231-232°C |
| 6 | Pen | Me | Me | Tet | O | O | 70 %<br>178-179°C |
| 7 | Ph | Me | Me | Tet | O | O | 85 %<br>272-273°C |
| 8 | 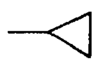 | Me | Me | Tet | O | O | 92 %<br>245-246°C |
| 9 | 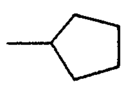 | Me | Me | Tet | O | O | 87 %<br>207-208°C |
| 10 | -$CH_2Ph$ | Me | Me | Tet | O | O | 43 %<br>150-151°C |
| 11 | -$(CH_2)_3Ph$ | Me | Me | Tet | O | O | 63 %<br>173-174°C |
| 12 | -$CH_2CHCH_3$<br>Ph | Me | Me | Tet | O | O | 51 %<br>155-157°C |
| 13 | Bu | Me | Me | Tet | S | O | 37 %<br>191-192°C |
| 14 | -$(CH_2)_4Ph$ | Me | Me | Tet | O | O | 66 %<br>93-95°C |

Table 3a (continued)

| Working Example No. | $R^1$ | $R^2$ | $R^3$ | $R^5$ | Y | Z | Yield, MP. |
|---|---|---|---|---|---|---|---|
| 15 | iso-Bu | Me | Me | Tet | O | O | 79 % 130-131°C |
| 16 | Hep | Me | Me | Tet | O | O | 73 % 143-144°C |
| 17 | -SBu | Me | Me | Tet | O | S | 29 % 177-178°C |
| 18 | -SBu | Me | Me | Tet | S | O | 23 % 201-202°C |
| 19 | -SPr | Me | Me | Tet | O | S | 62 % 210-211°C |
| 20 | -SPr | Me | Me | Tet | S | O | 18 % 190-191°C |
| 21 | Pr | Pr | Et | Tet | O | O | 40 % 149-150°C |
| 22 | Bu | Pr | Et | Tet | O | O | 67 % 150-151°C |
| 23 | Bu | Pr | Pr | Tet | O | O | 84 % 140-141°C |
| 24 | Bu | Me | Pr | Tet | O | O | 51 % 179-180°C |
| 25 | -SBu | Pr | Pr | Tet | O | O | 74 % 95-96°C |
| 26 | -SBu | Pr | Et | Tet | O | O | 72 % 83-85°C |
| 27 | Bu | Bu | Bu | Tet | O | O | 82 % 124-125°C |
| 28 | Bu | Bu | Pr | Tet | O | O | 91 % 165-166°C |

Table 3a (continued)

| Working Example No. | $R^1$ | $R^2$ | $R^3$ | $R^5$ | Y | Z | Yield, MP. |
|---|---|---|---|---|---|---|---|
| 29 | Bu | Me | Bu | Tet | O | O | 73 % 175-176°C |
| 30 | $-CH_2CH=CHCH_2CH_3$ | Pr | Pr | Tet | O | O | 60 % 203-204°C |
| 31 | Bu | Et | Et | Tet | O | O | 82 % 178-179°C |
| 32 | Dec | Me | Me | COOH | O | O | 79 % 128-129°C |
| 33 | SBu | Me | Me | COOH | O | O | 94 % 156-157°C |
| 34 | SBu | Me | Me | Tet | O | O | 24 % 183-185°C |
| 35 | OEt | Et | Et | Tet | O | O | 76 % 172-173°C |

<u>Table 3b</u>

| Working Ex. No. | NMR ( $\delta$ ) | IR (Nujol) $cm^{-1}$ | E. Anal. (Calc'd/ Found) C(%),H(%),O(%) |
|---|---|---|---|
| 5 | ($CDCl_3$) 0.97(3H,t),1.74(2H,dt), 2.69(2H,t),3.30(3H,s), 3.55(3H,s),5.50(2H,s), 7.06(2H,d),7.13(2H,d), 7.28(1H,s),7.39-7.66(3H, m),7.98(1H,dd) | 1705<br>1660<br>1605 | $C_{24}H_{24}N_8O$<br>63.15;5.30;24.55<br>62.96;5.43;24.24 |
| 6 | (DMSO-$d_6$) 0.86(3H,m),1.20-1.44(4H, m),1.62-1.81(2H,m),2.68 (2H,t),3.26(3H,s),3.52(3H, s),5.48(2H,s),7.08(2H,d), 7.10(2H,d),7.37-7.67(3H, m),7.93(1H,d) | 1705<br>1655 | $C_{26}H_{28}N_8O_2$<br>64.45;5.82;23.12<br>64.73;5.83;23.25 |
| 7 | (DMSO-$d_6$) 3.36(3H,s),3.61(3H,s),5.64 (2H,s),6.96(2H,d),7.06(2H, d),7.43-7.71(9H,m),7.87(1H ,s) | 1700<br>1645 | $C_{27}H_{22}N_8O_2$<br>66.11;4.52;22.84<br>65.72;4.75;22.21 |
| 8 | (DMSO-$d_6$) 0.89-1.09(4H,m),2.08-2.24 (1H,m),3.22(3H,s),3.37(3H, s),5.66(2H,s),7.07(2H,d), 7.21(2H,d),7.49-7.61(4H,m) | 1705<br>1660<br>1640 | $C_{24}H_{22}N_8O_2$<br>63.43;4.88;24.65<br>63.24;5.00;24.42 |
| 9 | (DMSO-$d_6$) 1.51-1.89(4H,m),3.13-3.62 (5H,m),3.23(3H,s),3.43 (3H,s),5.61(2H,s),7.06(2H, d),7.12(2H,d),7.49-7.74 (4H,m) | 1700<br>1660<br>1640 | $C_{26}H_{26}N_8O_2$<br>64.72;5.43;23.22<br>64.54;5.50;22.82 |
| 10 | (DMSO-$d_6$) 3.22(3H,s),3.41(3H,s),4.12 (2H,s),5.58(2H,s),6.98- 7.30(9H,m),7.47-7.75(4H, m) | 1695<br>1630 | $C_{28}H_{24}N_8O_2$<br>66.65;4.79;22.21<br>66.30;4.69;22.05 |

Table 3b (continued)

| Working Ex. No. | NMR (δ) | IR (Nujol) $cm^{-1}$ | E. Anal. (Calc'd/ Found) C(%),H(%),O(%) |
|---|---|---|---|
| 11 | (DMSO-$d_6$) 1.83(2H,m),2.60(2H,t),2.68 (2H,t),3.23(3H,s),3.43(3H, s),5.52(2H,s),7.07(5H,s), 7.16-7.31(6H,m) | 1705 1660 1650 1605 | $C_{30}H_{28}N_8O_2$ 67.65;5.30;21.04 67.58;5.30;20.87 |
| 12 | (DMSO-$d_6$) 1.16(3H,d),2.96(2H,d),3.21 (3H,s),3.44(3H,s),5.43(2H, dd),7.06(5H,s),7.12-7.32 (4H,m),7.27-7.44(4H,m) | 1700 1630 1605 | $C_{30}H_{28}N_8O_2$ 67.65;5.30;21.04 67.44;5.30;20.89 |
| 13 | (DMSO-$d_6$) 0.83(3H,t),1.19-1.39(2H, m),1.32-1.64(2H,m),2.71 (2H,t),3.68(3H,s),3.87(3H, s),5.62(2H,s),7.06(4H,d), 7.15(2H,d),7.46-7.74(4H,m) | 1690 1650 1600 | $C_{25}H_{26}N_8OS$ 61.71;5.39;23.03 61.76;5.41;22.84 |
| 14 | (DMSO-$d_6$) 1.52-1.70(4H,m),2.64-2.79 (4H,m),3.23(3H,s),3.42 (3H,s),5.56(2H,s),7.00- 7.31(9H,m),7.44-7.72 (4H,m) | 1700 1655 1600 | $C_{31}H_{30}N_8O_2$ · $1/2H_2O$ 67.01;5.62;20.17 67.15;5.76;20.32 |
| 15 | (DMSO-$d_6$) 0.83(3H,s),0.86(3H,s),1.88 -2.10(1H,m),2.55(2H,d), 3.23(3H,s),3.44(3H,s),5.57 (2H,s),7.06(2H,d),7.13(2H, d),7.53(1H,t),7.59-7.74 (3H,m) | 1700 1660 1605 | $C_{25}H_{26}N_8O_2$ · $1/2H_2O$ 62.62;5.68;23.37 62.62;5.76;23.55 |
| 16 | (DMSO-$d_6$) 0.82(3H,t),1.12-1.36(14H, m),1.48-1.65(2H,m),2.71 (2H,t),3.24(3H,s),3.44(3H, s),5.61(2H,s),7.18-7.61 (7H,m),7.73(1H,dd) | 1705 1655 1600 | $C_{28}H_{32}N_8O_2$ 65.61;6.29;21.86 65.77;6.26;21.74 |

Table 3b (continued)

| Working Ex. No. | NMR ( δ ) | IR (Nujol) $cm^{-1}$ | E. Anal. (Calc'd/ Found) C(%),H(%),O(%) |
|---|---|---|---|
| 17 | ($DMSO-d_6$) 0.84(3H,t),1.36(2H,m),1.70 (2H,m),3.16(2H,t),3.52(3H, s),3.54(3H,s),5.30(2H,s), 7.07(2H,d),7.36(2H,d),7.50 -7.74(4H,m) | 1670<br>1575 | $C_{25}H_{26}N_8OS_2$<br>57.89;5.05;21.60<br>58.06;5.15;21.29 |
| 18 | ($DMSO-d_6$) 0.89(3H,t),1.30-1.49(2H, m),1.60-1.78(2H,m),3.28 (2H,t),3.67(3H,s),3.87(3H, s),5.46(2H,s),7.07(2H,d), 7.12(2H,d),7.47-7.74(4H,m) | 1685<br>1600 | $C_{25}H_{26}N_8OS_2$ · $1/5H_2O$<br>57.49;5.09;21.46<br>57.25;5.12;21.15 |
| 19 | ($DMSO-d_6$) 0.95(3H,t),1.62-1.81(2H, m),3.26(2H,t),3.67(3H,s), 3.87(3H,s),5.47(2H,s), 7.08(2H,d),7.22(2H,d), 7.48-7.74(4H,m) | 1670<br>1600 | $C_{24}H_{24}N_8OS_2$<br>57.12;4.79;22.20<br>57.26;4.73;21.93 |
| 20 | ($DMSO-d_6$) 0.93(3H,t),1.65-1.85(2H, m),3.14(2H,t),3.52(3H,s), 3.54(3H,s),5.29(2H,s), 7.06(2H,d),7.37(2H,d), 7.50-7.75(4H,m) | 1690<br>1580 | $C_{24}H_{24}N_8OS_2$<br>57.12;4.79;22.20<br>57.25;4.87;21.71 |
| 21 | ($DMSO-d_6$) 0.96(6H,t),1.18(3H,t), 1.64-1.91(4H,m),2.66(2H, t),4.00(2H,t),4.02(2H,q), 5.50(2H,s),7.07(2H,d), 7.13(2H,d),7.39-7.64(3H, m),7.92(1H,dd) | 1695<br>1660<br>1600 | $C_{27}H_{30}N_8O_2$ · $1/2H_2O$<br>63.89;6.16;22.08<br>63.66;4.43;22.12 |

Table 3b (continued)

| Working Ex. No. | NMR (δ) | IR (Nujol) $cm^{-1}$ | E. Anal. (Calc'd/Found) C(%),H(%),O(%) |
|---|---|---|---|
| 22 | ($CDCl_3$) 0.90(3H,t),0.95(3H,t), 1.15(3H,t),1.27-1.50(2H, m),1.53-1.89(4H,m),2.69 (2H,t),3.83-4.13(4H,m), 5.48(2H,s),7.05(2H,d) | 1695 1660 1600 | $C_{28}H_{32}N_8O_2$ 65.61;6.29;21.86 65.78;6.35;21.68 |
| 23 | ($DMSO-d_6$) 0.82(3H,t),0.86(3H,t),0.88 (3H,t),1.18-1.40(2H,m), 1.44-1.82(6H,m),2.66(2H, t),3.83(2H,t),3.95(2H,t), 5.56(2H,s),7.07(2H,d),7.15 (2H,d),7.46-7.73(4H,m) | 1695 1660 1600 | $C_{29}H_{34}N_8O_2$ 66.14;6.51;21.28 66.35;6.57;21.11 |
| 24 | ($CDCl_3$) 0.87(3H,t),0.91(3H,t), 1.30-1.49(2H,m),1.53-1.79 (4H,m),2.69(2H,t),3.52(3H, s),3.86(2H,t),5.48(2H,s), 7.04(2H,d),7.14(2H,d),7.36 -7.66(3H,m),7.93(1H,dd) | 1700 1655 1605 | $C_{27}H_{30}N_8O_2$ 65.04;6.06;22.47 65.27;6.05;22.38 |
| 25 | ($DMSO-d_6$) 0.86(3H,t),0.87(3H,t), 1.35(2H,m),1.49-1.79(4H, m),3.22(2H,t),3.82(2H,t), 3.95(2H,t),5.42(2H,s),7.07 (2H,d),7.21(2H,d),7.48 -7.73(4H,m) | 1700 1655 1600 | $C_{29}H_{34}N_8O_2S$ 62.34;6.13;20.06 61.81;5.92;20.21 |
| 26 | ($CDCl_3$) 0.95(3H,t),0.96(3H,t), 1.11(3H,t),1.38-1.89(6H, m),3.29(2H,t),3.89-4.13 (4H,m),5.40(2H,s),7.14(2H, d),7.28(2H,d),7.38-7.66 (3H,m),8.04(1H,d) | 1695 1655 1595 | $C_{28}H_{32}N_8O_2S$ 61.74;5.92;20.57 61.63;5.70;20.24 |

Table 3b (continued)

| Working Ex. No. | NMR (δ) | IR (Nujol) $cm^{-1}$ | E. Anal. (Calc'd/ Found) C(%),H(%),O(%) |
|---|---|---|---|
| 27 | ($CDCl_3$) 0.89(3H,t),0.91(3H,t), 0.95(3H,t),1.21-1.84(12H, m),2.69(2H,t),3.89(2H,t), 4.06(2H,t),5.48(2H,s),7.05 (2H,d),7.17(2H,d),7.35-7.69(3H,m),7.96(1H,dd) | 1690 1645 1600 | $C_{31}H_{38}N_8O_2$ 67.13;6.91;20.20 67.27;6.94;20.10 |
| 28 | ($CDCl_3$) 0.88(3H,t),0.91(3H,t), 0.95(3H,t),1.28-1.83(10H, m),2.70(2H,t),3.86(2H,t), 4.07(2H,t),5.49(2H,s),7.07 (2H,d),7.13(2H,d),7.37-7.64(3H,m),7.98(1H,dd) | 1690 1650 1595 | $C_{30}H_{36}N_8O_2$ 66.65;6.71;20.73 66.73;6.82;20.42 |
| 29 | (DMSO-$d_6$) 0.82(3H,t),0.90(3H,t),1.19 -1.63(8H,m),2.66(2H,t), 3.42(3H,s),3.87(2H,t), 5.56(2H,s),7.06(2H,d),7.14 (2H,d),7.47-7.73(4H,m) | 1700 1655 1605 | $C_{28}H_{32}N_8O_2$ 65.61;6.29;21.86 65.89;6.37;21.77 |
| 30 | (DMSO-$d_6$) 0.86(3H,t),0.89(6H,t),1.38 -1.83(6H,m),2.23(2H,dt), 3.83(2H,t),3.96(2H,t), 5.63(2H,s),6.61(1H,d),6.80 -6.97(1H,m),7.06(2H,d), 7.18(2H,d),7.47-7.72(4H,m) | 1685 1655 1590 | $C_{30}H_{34}N_8O_2$ 66.89;6.36;20.80 67.03;6.43;20.78 |
| 31 | ($CDCl_3$) 0.82(3H,t),1.12(3H,t), 1.23(3H,t),1.28(2H,m), 1.52(2H,m),2.66(2H,t), 3.92(2H,q),4.02(2H,q), 5.56(2H,s),7.07(2H,d), 7.15(2H,d),7.47-7.74(4H, m) | 1695 1660 1605 | $C_{27}H_{30}N_8O_2$ 65.04;6.06;22.47 65.32;6.18;22.23 |

Table 3b (continued)

| Working Ex. No. | NMR (δ) | IR (Nujol) $cm^{-1}$ | E. Anal. (Calc'd/ Found) C(%),H(%),O(%) |
|---|---|---|---|
| 32 | (DMSO-$d_6$) 0.82(3H,t),1.12-1.36(14H, m),1.48-1.65(2H,m),2.71 (2H,t),3.24(3H,s),3.44(3H, s),5.61(2H,s),7.18-7.61 (7H,m),7.73(1H,dd) | 1705<br>1670<br>1600 | $C_{31}H_{38}N_4O_4$<br>70.16;7.22;10.56<br>70.36;7.18;10.61 |
| 33 | (DMSO-$d_6$) 0.89(3H,t),1.38(2H,m), 1.67(2H,m),3.24(3H,s), 3.45(3H,s),5.48(2H,s), 7.27-7.50(6H,m),7.57(1H, dt),7.72(1H,dd) | 3170<br>1700<br>1660<br>1630 | $C_{25}H_{26}N_4O_4S$<br>62.74;5.48;11.71<br>62.91;5.56;11.58 |
| 34 | ($CDCl_3$) 0.95(3H,t),1.46(2H,m), 1.75(2H,m),3.30(3H,s), 3.31(2H,t),3.55(3H,s), 5.42(2H,s),7.11-7.66(7H, m),8.08(1H,dd) | 1695<br>1660<br>1600 | $C_{25}H_{26}N_8O_2S$<br>59.74;5.21;22.29<br>59.98;5.16;22.05 |
| 35 | 1.14(3H,t),1.30(3H,t), 1.46(3H,t),3.39(2H,q), 4.07(2H,q),4.58(2H,q), 5.23(2H,s),7.13(2H,d), 7.29(2H,d),7.39-7.64(3H, m),7.96-8.04(1H,m) | 1695<br>1660<br>1610 | $C_{25}H_{26}N_8O_3$<br>61.72;5.39;23.03<br>61.68;5.32;22.85 |

Pharmaceutical Examples

The compounds (I) of the present invention are employed, for example, when used as agents for treating circulatory system diseases such as hypertension, heart diseases, strokes and the like, in the following formulations.

1. Capsule

| | | |
|---|---|---|
| (1) | 8-n-Butyl-7-(2'-((1H-tetrazole-5-yl)biphenyl-4-yl)methyl)-1,3-dimethylxanthine | 10 mg |
| (2) | Lactose | 90 mg |
| (3) | Microcrystalline cellulose | 70 mg |
| (4) | Magnesium stearate | 10 mg |
| | One capsule | 180 mg |

The ingredients (1), (2), and (3) and a half of the ingredient (4) were blended together and granulated. To this mixture was added the remaining half of the ingredient (4) and distributed into gelatine capsules.

2. Tablet

| | | |
|---|---|---|
| (1) | 8-n-Butyl-7-(2'-((1H-tetrazole-5-yl)biphenyl-4-yl)methyl)-1,3-dimethylxanthine | 10 mg |
| (2) | Lactose | 35 mg |
| (3) | Maize starch | 150 mg |
| (4) | Microcrystalline cellulose | 30 mg |
| (5) | Magnesium stearate | 5 mg |
| | One tablet | 230 mg |

Two third each of the ingredients (1), (2), (3) and (4) and a half of the ingredient (5) were blended together and granulated. To these granules were added the remaining ingredients (4) and (5) and then compressed to form tablets.

3. Injection

| | | |
|---|---|---|
| (1) | 8-n-Butyl-7-(2'-((1H-tetrazole-5-yl)biphenyl-4-yl)methyl)-1,3-dimethylxanthine • sodium salt | 10 mg |
| (2) | Inositol | 100 mg |
| (3) | Benzyl alcohol | 20 mg |
| | One ampule | 130 mg |

The ingredients (1), (2) and (3) were dissolved in distilled water for injection to a total volume of two ml and distributed into ampules. Total processes were carried out under sterile conditions.

Biological Test

Inhibitory Effect on Binding of Angiotensin II to Angiotensin Receptor

Method

Inhibitory assay for the binding of angiotensin II to angiotensin receptor was conducted by modifying a method as described by Douglas et al. (Endocrinology, 102, 685-696 (1978)).

Angiotensin II receptor membrane fractions were prepared from bovine adrenal cortex.

The compound (I) of the present invention ($10^{-9}$-$3x10^{-5}$M) and $^{125}$I-angiotensin II ($^{125}$I-AII) (1.8kBq/50μl) were added to the A II receptor membrane fraction and incubated at room temperature for one hour. The bound and free $^{125}$I-AII were separated by a filter (Whatman GF/B filter) each other. The radioactivity of $^{125}$I-AII bound on the receptor was measured.

The results of the test compounds are shown in Table 4.

TABLE 4

| Compound No. | Inhibitory Activity against Binding of Angiotensin II $IC_{50}$ (M) |
|---|---|
| 1 | $2.8 \times 10^{-7}$ |
| 2 | $4.9 \times 10^{-8}$ |
| 4 | $6.2 \times 10^{-8}$ |
| 5 | $1.7 \times 10^{-7}$ |
| 7 | $6.5 \times 10^{-8}$ |
| 8 | $6.0 \times 10^{-7}$ |
| 9 | $9.4 \times 10^{-7}$ |
| 10 | $3.4 \times 10^{-6}$ |
| 12 | $3.3 \times 10^{-7}$ |
| 13 | $4.9 \times 10^{-6}$ |
| 14 | $3.3 \times 10^{-7}$ |
| 15 | $1.5 \times 10^{-7}$ |
| 17 | $7.6 \times 10^{-7}$ |
| 19 | $1.5 \times 10^{-6}$ |
| 20 | $1.5 \times 10^{-6}$ |
| 21 | $5.6 \times 10^{-7}$ |
| 22 | $8.4 \times 10^{-7}$ |
| 23 | $1.1 \times 10^{-6}$ |
| 25 | $5.6 \times 10^{-7}$ |
| 26 | $2.0 \times 10^{-6}$ |

TABLE 4 (continued)

| Compound No. | Inhibitory Activity against Binding of Angiotensin II $IC_{50}$ (M) |
|---|---|
| 27 | $2.1 \times 10^{-8}$ |
| 28 | $1.9 \times 10^{-7}$ |
| 29 | $1.0 \times 10^{-8}$ |
| 30 | $3.8 \times 10^{-7}$ |
| 31 | $1.1 \times 10^{-7}$ |
| 33 | $2.8 \times 10^{-7}$ |
| 34 | $1.3 \times 10^{-7}$ |
| 35 | $1.1 \times 10^{-8}$ |

$IC_{50}$: concentration of the test compound which inhibits 50% of the A II binding.

It is understood that the preceding representative examples may be varied within the scope of the present invention by one skilled in the art to achieve essentially the same results.

As many widely different embodiments of this invention may be made without departing from the spirit and scope thereof, it is to be understood that this invention is not limited to the specific embodiments thereof except as defined in the appended claims.

## Claims

1. A compound of the formula:

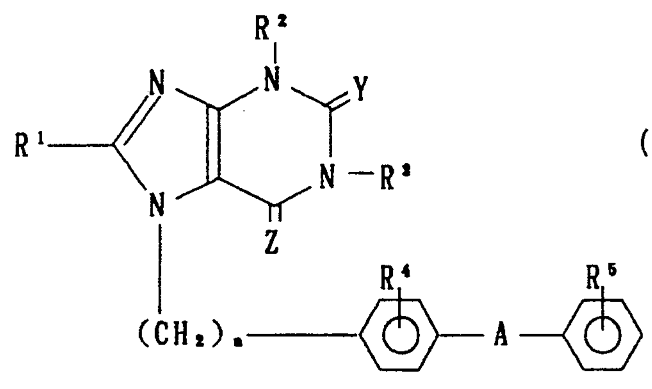

(I)

wherein $R^1$, which may be optionally bound through a hetero atom, is a hydrocarbon residue which may be substituted;
one of $R^2$ and $R^3$ is a hydrocarbon residue which may be substituted, and the other is hydrogen or a hydrocarbon residue which may be substituted;
$R^4$ is hydrogen, halogen or nitro;

$R^5$ is a residue capable of forming an anion or a residue convertible into the anion;
A is a direct bond or a spacer having atomic length of two or less;
n is an integer of 1 or 2; and
Y and Z, which may be the same or different, are each independently selected from an oxygen atom or a sulfur atom;
and the pharmaceutically acceptable salts thereof.

2. A compound according to claim 1, wherein $R^1$ is lower alkyl of 1 to about 8 carbon atoms, lower alkenyl of 2 to about 8 carbon atoms, lower alkynyl of 2 to about 8 carbon atoms, alicyclic hydrocarbon residue of 3 to about 8 carbon atoms or aromatic hydrocarbon residue of 6 to about 12 carbon atoms which may be optionally bound to a xanthine nucleus through a hetero atom selected from S, O, and NR wherein R is hydrogen or a hydrocarbon residue and optionally substituted with hydroxyl, lower ($C_1$-$_4$) alkoxy, lower ($C_1$-$_4$) alkyl, halogen, nitro, optionally substituted amino, acyloxy, or phenyl which may be optionally substituted with halogen, nitro, lower ($C_1$-$_4$) alkoxy, or lower ($C_1$-$_4$) alkyl.

3. A compound according to claim 1, wherein $R^1$ may be bound to a xanthine nucleus through a sulfur or oxygen atom, and is lower alkyl of 1 to about 8 carbon atoms, lower alkyl of 1 to about 4 carbon atoms substituted with phenyl which may be optionally substituted with halogen, nitro, lower ($C_1$-$_4$) alkoxy, or lower ($C_1$-$_4$) alkyl at an optional position on the phenyl ring, or phenyl which may be optionally substituted with halogen, nitro, lower ($C_1$-$_4$) alkoxy, or lower ($C_1$-$_4$) alkyl at an optional position on the phenyl ring.

4. A compound according to claim 1, wherein $R^1$ is n-propyl, n-butyl, isobutyl, n-pentyl, n-heptyl, n-decyl, n-pentenyl, cyclopropyl, cyclopentyl, cyclohexyl, phenyl, benzyl, 3-phenylpropyl, 2-phenylpropyl, 4-phenylbutyl, ethoxy, n-propylthio, or n-butylthio.

5. A compound according to claim 1, wherein $R^2$ is lower alkyl of 1 to about 4 carbon atoms, lower alkenyl of 2 to about 4 carbon atoms, lower alkynyl of 2 to about 4 carbon atoms, alicyclic hydrocarbon residue of 3 to about 6 carbon atoms, or aromatic hydrocarbon residue of about 6 carbon atoms.

6. A compound according to claim 1, wherein $R^3$ is lower alkyl of 1 to about 4 carbon atoms, lower alkenyl of 2 to about 4 carbon atoms, lower alkynyl of 2 to about 4 carbon atoms, alicyclic hydrocarbon residue of 3 to about 6 carbon atoms, or aromatic hydrocarbon residue of about 6 carbon atoms.

7. A compound according to claim 1, wherein $R^2$ is hydrocarbon residue substituted with hydroxyl, lower ($C_1$-$_4$) alkoxy, lower ($C_1$-$_4$) alkyl, halogen, nitro, optionally substituted amino, acyloxy, or phenyl which may be optionally substituted with halogen, nitro, lower ($C_1$-$_4$) alkoxy, or lower ($C_1$-$_4$) alkyl at an optional position on the phenyl ring.

8. A compound according to claim 1, wherein $R^3$ is hydrocarbon residue substituted with hydroxyl, lower ($C_1$-$_4$) alkoxy, lower ($C_1$-$_4$) alkyl, halogen, nitro, optionally substituted amino, acyloxy, or phenyl which may be optionally substituted with halogen, nitro, lower ($C_1$-$_4$) alkoxy, or lower ($C_1$-$_4$) alkyl at an optional position on the phenyl ring.

9. A compound according to claim 1, wherein $R^2$ is lower ($C_1$-$_4$) alkyl of 1 to about 4 carbon atoms which may be optionally substituted with hydroxyl, lower ($C_1$-$_4$) alkoxy, optionally substituted amino, or acyloxy, lower alkyl of 1 to about 4 carbon atoms substituted with phenyl which may be optionally substituted with halogen, nitro, lower ($C_1$-$_4$) alkoxy, or lower ($C_1$-$_4$) alkyl at an optional position on the phenyl ring, or phenyl which may be optionally substituted with halogen, nitro, lower ($C_1$-$_4$) alkoxy, or lower ($C_1$-$_4$) alkyl at an optional position on the phenyl ring.

10. A compound according to claim 1, wherein $R^3$ is lower ($C_1$-$_4$) alkyl of 1 to about 4 carbon atoms which may be optionally substituted with hydroxyl, lower ($C_1$-$_4$) alkoxy, optionally substituted amino, or acyloxy, lower alkyl of 1 to about 4 carbon atoms substituted with phenyl which may be optionally substituted with halogen, nitro, lower ($C_1$-$_4$) alkoxy, or lower ($C_1$-$_4$) alkyl at an optional position on the phenyl ring, or phenyl which may be optionally substituted with halogen, nitro, lower ($C_1$-$_4$) alkoxy, or lower ($C_1$-$_4$) alkyl at an optional position on the phenyl ring.

**11.** A compound according to claim 1, wherein $R^2$ is lower alkyl of 1 to about 4 carbon atoms.

**12.** A compound according to claim 1, wherein $R^3$ is lower alkyl of 1 to about 4 carbon atoms.

**13.** A compound according to claim 1, wherein $R^2$ is methyl, ethyl, n-propyl, or n-butyl.

**14.** A compound according to claim 1, wherein $R^3$ is methyl, ethyl, n-propyl, or n-butyl.

**15.** A compound according to claim 1, wherein $R^4$ is hydrogen, halogen, or nitro.

**16.** A compound according to claim 1, wherein $R^4$ is hydrogen.

**17.** A compound according to claim 1, wherein $R^5$ is carboxyl, lower ($C_{1-4}$) alkoxycarbonyl, cyano, tetrazolyl, trifluoromethanesulfonic amide, phosphoric acid, or sulfonic acid.

**18.** A compound according to claim 1, wherein $R^5$ is carboxyl or tetrazolyl.

**19.** A compound according to claim 1, wherein $R^5$ is tetrazolyl.

**20.** A compound according to claim 1, wherein $R^5$ is in the ortho position.

**21.** A compound according to claim 1, wherein the group A is a spacer having a two or less divalent atom chain wherein the constituent number of atoms in a straight chain is 1 or 2 and which may be branched.

**22.** A compound according to claim 1, wherein the group A is a spacer selected from -C(=O)-, -O-, -S-, -NH-, -C(=O)-NH-, $-O-CH_2-$, $-S-CH_2-$, or -CH=CH-.

**23.** A compound according to claim 1, wherein the group A is a direct bond between a phenylene group and a phenyl group.

**24.** A compound according to claim 1, wherein the groups Y and Z are each an oxygen atom.

**25.** A compound according to claim 1, wherein n is 1.

**26.** A compound according to claim 1 or a pharmaceutically acceptable salt thereof, which is 8-n-butyl-7-(2'-((1H-tetrazole-5-yl)biphenyl-4-yl)methyl)-1,3-dimethylxanthine.

**27.** A compound according to claim 1 or a pharmaceutically acceptable salt thereof, which is 8-n-propyl-7-(2'-((1H-tetrazole-5-yl)biphenyl-4-yl)methyl 1,3-dimethylxanthine.

**28.** A compound according to claim 1 or a pharmaceutically acceptable salt thereof, which is 8-n-pentyl-7-(2'-((1H-tetrazole-5-yl)biphenyl-4-yl)methyl 1,3-dimethylxanthine.

**29.** A compound according to claim 1 or a pharmaceutically acceptable salt thereof, which is 8-cyclopropyl-7-(2'-((1H-tetrazole-5-yl)biphenyl-4-yl) methyl)-1,3-dimethylxanthine.

**30.** A pharmaceutical composition for antagonizing angiotensin II which comprises a therapeutically effective amount of a compound according to claim 1 or a pharmaceutically acceptable salt thereof in admixture with a pharmaceutical acceptable carrier, excipient or diluent.

**31.** A use of a compound according to claim 1 or a pharmaceutically acceptable salt thereof for the manufacture of a medicament for antagonizing angiotensin II.

**32.** A method for producing a compound of the formula (I):

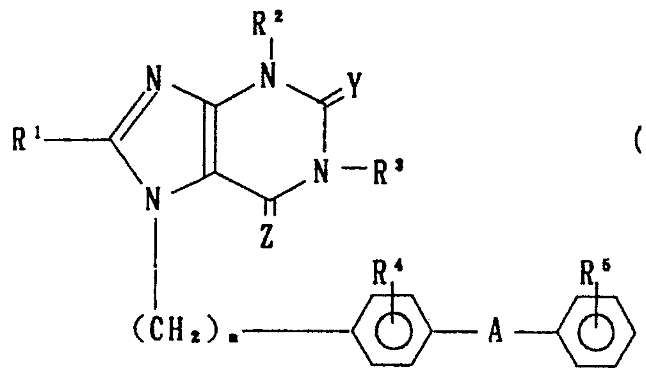

(I)

wherein $R^1$, which may be optionally bound through a hetero atom, is a hydrocarbon residue which may be substituted;

one of $R^2$ and $R^3$ is a hydrocarbon residue which may be substituted, and the other is hydrogen or a hydrocarbon residue which may be substituted;

$R^4$ is hydrogen, halogen or nitro;

$R^5$ is a residue capable of forming an anion or a residue convertible into the anion;

A is a direct bond or a spacer having atomic length of two or less;

n is an integer of 1 or 2; and

Y and Z, which may be the same or different, are each independently selected from an oxygen atom or a sulfur atom;

and the pharmaceutically acceptable salts thereof, which comprises reacting a compound of the formula (II):

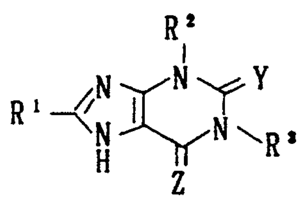

(II)

wherein $R^1$, $R^2$, $R^3$, Z, and Y have the above-defined meanings, with a compound of the formula (III):

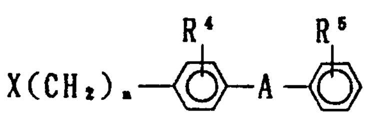

(III)

wherein $R^4$, $R^5$, A, and n have the above-defined meanings and X is halogen, and, (i) if desired, converting a compound of the formula (I) wherein $R^5$ is cyano, and $R^1$, $R^2$, $R^3$, $R^4$, Z, Y, A, and n have the above-defined meanings, into a compound of the formula (I) wherein $R^5$ is tetrazolyl and $R^1$, $R^2$, $R^3$, $R^4$, Z, Y, A, and n have the above-defined meanings, or (ii) if desired, converting a compound of the formula (I) wherein $R^5$ is an ester, and $R^1$, $R^2$, $R^3$, $R^4$, Z, Y, A, and n have the above-defined meanings, into a compound of the formula (I) wherein $R^5$ is carboxyl and $R^1$, $R^2$, $R^3$, $R^4$, Z, Y, A, and n have the above-defined meanings, or (iii) if desired, converting a compound of the formula (I) wherein $R^5$ is optionally protected tetrazolyl, and $R^1$, $R^2$, $R^3$, $R^4$, Z, Y, A, and n have the above-defined meanings, into a compound of the formula (I) wherein $R^5$ is tetrazolyl and $R^1$, $R^2$, $R^3$, $R^4$, Z, Y, A, and n have the above-defined meanings, and if desired, converting a compound of the formula (I) into a pharmaceutically acceptable salt thereof.